(19) **Europäisches Patentamt / European Patent Office / Office européen des brevets**

(11) **EP 4 591 869 A1**

(12) **EUROPEAN PATENT APPLICATION**
published in accordance with Art. 153(4) EPC

(43) Date of publication:
**30.07.2025 Bulletin 2025/31**

(21) Application number: **23868141.5**

(22) Date of filing: **15.09.2023**

(51) International Patent Classification (IPC):
**A61K 35/32** (2015.01)   **A61P 19/08** (2006.01)
**A61P 43/00** (2006.01)   **C12N 5/0775** (2010.01)

(52) Cooperative Patent Classification (CPC):
**A61K 35/32; A61P 19/08; A61P 43/00; C12N 5/06**

(86) International application number:
**PCT/JP2023/033663**

(87) International publication number:
**WO 2024/063020 (28.03.2024 Gazette 2024/13)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **20.09.2022 JP 2022149224**

(71) Applicants:
• **Two Cells Co., Ltd**
  **Hiroshima-shi, Hiroshima 734-0001 (JP)**
• **OSAKA UNIVERSITY**
  **Suita-shi**
  **Osaka 565-0871 (JP)**

(72) Inventors:
• **SAKAUE, Morito**
  **Hiroshima-shi, Hiroshima 734-0001 (JP)**
• **MATSUMOTO, Masaya**
  **Hiroshima-shi, Hiroshima 734-0001 (JP)**
• **NAGAYA, Masahiro**
  **Hiroshima-shi, Hiroshima 734-0001 (JP)**
• **KAITO, Takashi**
  **Suita-shi, Osaka 565-0871 (JP)**
• **KODAMA, Joe**
  **Suita-shi, Osaka 565-0871 (JP)**
• **HIRAI, Hiromasa**
  **Suita-shi, Osaka 565-0871 (JP)**

(74) Representative: **Plasseraud IP**
  **104 Rue de Richelieu**
  **CS92104**
  **75080 Paris Cedex 02 (FR)**

(54) **INTERVERTEBRAL DISC THERAPEUTIC AGENT**

(57) Provided is an intervertebral disc therapeutic agent that exhibits favorable post-transplantation engraftment. The intervertebral disc therapeutic agent contains a scaffold-free artificial tissue in which synovium-derived mesenchymal stem cells have formed a three-dimensional structure.

FIG. 1

LATERAL RADIOGRAPH

EP 4 591 869 A1

## Description

Technical Field

The present invention relates to an intervertebral disc therapeutic agent.

Background Art

Damage to an intervertebral disc is likely to have a severe effect on life when accompanied by pain from the back to the waist. In recent years, regenerative medicine by means of cell transplantation is applied to such damage to an intervertebral disc.

As cells applicable to regenerative medicine, for example, Patent Literature 1 proposes a scaffold-free selforganized 3D artificial tissue which has tissue strength that allows for clinical application. Further, Non-Patent Literature 1 indicates a therapeutic effect on intervertebral disc damage, which is yielded by transplantation of a tissue engineered construct (TEC) that is prepared by a method disclosed in Patent Literature 1 with use of rat adiposederived mesenchymal stem cells.

Citation List

[Patent Literature]

[Patent Literature 1]
Pamphlet of International Publication No. WO 2005/012512

[Non-patent Literature]

[Non-patent Literature 1]
H. Ishiguro et. al., Acta Biomaterialia 87, 118-129, 2019

Summary of Invention

Technical Problem

However, there is room for improvement in the method disclosed in Non-Patent Literature 1 in terms of engraftment of a transplanted tissue engineered construct. An object of an aspect of the present invention is to provide an intervertebral disc therapeutic agent that exhibits favorable post-transplantation engraftment.

Solution to Problem

In order to solve the above problem, an intervertebral disc therapeutic agent in accordance with an embodiment of the present invention contains a scaffold-free artificial tissue in which synovium-derived mesenchymal stem cells have formed a three-dimensional structure.

Advantageous Effects of Invention

An aspect of the present invention can provide an intervertebral disc therapeutic agent that exhibits favorable post-transplantation engraftment.

Brief Description of Drawings

Fig. 1 is a diagram illustrating intervertebral disc height (DHI) evaluation in Examples.
Fig. 2 is a diagram illustrating endplate damage score (CT score) evaluation using CT in Examples.
Fig. 3 is a diagram showing radiographs of surgery sites of rat groups used in Examples.
Fig. 4 is a diagram showing results of DHI and CT scores which were evaluated in Examples.
Fig. 5 is a diagram showing a result of vimentin staining in Examples.

Description of Embodiments

The following description will discuss embodiments of the present invention. Note, however, that the present invention is not limited to these embodiments. Note that the numerical ranges "A to B" herein each mean "not less than A and not more than B", unless otherwise noted herein.

[Intervertebral disc therapeutic agent]

An intervertebral disc therapeutic agent in accordance with an aspect of the present invention contains a scaffold-free artificial tissue in which synovium-derived mesenchymal stem cells have formed a three-dimensional structure.

The present inventors found that a scaffold-free artificial tissue in which synovium-derived mesenchymal stem cells had formed a three-dimensional structure was effective in intervertebral disc treatment. The present inventors also found that the scaffold-free artificial tissue in which synovium-derived mesenchymal stem cells had formed a three-dimensional structure exhibited favorable engraftment to a transplantation site, and also remained at the transplantation site for a long period of time. The intervertebral disc therapeutic agent that contains a scaffold-free artificial tissue in which synovium-derived mesenchymal stem cells have formed a three-dimensional structure thus has an excellent therapeutic effect and also exhibits excellent durability of the therapeutic effect.

The intervertebral disc therapeutic agent can be used for treatment of a damaged site or a defective site of a intervertebral disc. The intervertebral disc therapeutic agent treats damage or deficiency of the intervertebral disc, when administered to a site that requires treatment. When the intervertebral disc therapeutic agent is administered to a damaged site or a defective site of an intervertebral disc, regeneration of the intervertebral disc at an administration site is promoted. Thus, the intervertebral disc therapeutic agent can treat damage or deficiency of the intervertebral disc.

In intervertebral disc treatment using the intervertebral disc therapeutic agent, a method of administering the intervertebral disc therapeutic agent is not particularly limited. The intervertebral disc therapeutic agent can be administered by, for example, transplanting the intervertebral disc therapeutic agent to a treatment site, or injecting, to a treatment site, an injection which contains the intervertebral disc therapeutic agent. It is possible to administer, to the treatment site, the intervertebral disc therapeutic agent alone or the intervertebral disc therapeutic agent in combination with another therapeutic agent.

(Synovium-derived mesenchymal stem cells)

"Mesenchymal stem cells" herein mean somatic stem cells that differentiate into mesenchymal tissue. The mesenchymal stem cells also include: cells that are obtained by isolating, from mesenchymal stem cells, cells that have an additional specific property; cells that are obtained by giving some stimulus, such as cytokine stimulus, to mesenchymal stem cells; and cells that are obtained by gene modification to mesenchymal stem cells. The mesenchymal stem cells include, for example, MUSE cells, MAPC cells, and SP-1 cells. The mesenchymal stem cells have proliferation potency and differentiation potency into osteocytes, chondrocytes, muscle cells, stromal cells, tenocytes, adipocytes, and the like. It is known that the mesenchymal stem cells are isolated not only from adult tissues of, for example, bone marrow, adipocytes, synoviocytes, alveolar bone, and periodontal ligament, but also from, for example, various cells of placentas, umbilical cords, umbilical cord blood, and fetus.

The synovium-derived mesenchymal stem cells that form an artificial tissue contained in the intervertebral disc therapeutic agent are stem cells that are contained in synovium, and are obtained from a synovial tissue by a known method. The synovium-derived mesenchymal stem cells have differentiation potency to differentiate into chondrocytes. The synovium-derived mesenchymal stem cells may be mesenchymal stem cells derived from synovium of a non-human animal such as a rat or a mouse, but it is preferable that the synovium-derived mesenchymal stem cells be human synovium-derived mesenchymal stem cells. When the intervertebral disc therapeutic agent is administered to a treatment site of a human, human synovium-derived mesenchymal stem cells exhibit better engraftment to the treatment site and exhibit excellent durability of the therapeutic effect. In addition, the human synovium-derived mesenchymal stem cells can suppress risks such as biological contamination of a human who administers the intervertebral disc therapeutic agent and mixing of an immunogenic substance.

The synovium-derived mesenchymal stem cells can be cells which are cultured by a conventionally known method. The mesenchymal stem cells are preferably serum-free cultured cells or low serum cultured cells, and more preferably serum-free cultured cells. Further, when the intervertebral disc therapeutic agent is administered to a human treatment site, the serum-free cultured synovium-derived mesenchymal stem cells exhibit better engraftment to the treatment site and exhibit excellent durability of the therapeutic effect.

In addition, in a case where the synovium-derived mesenchymal stem cells are serum-free cultured, cell culture ingredients are known. In other words, since serum is derived from a natural ingredient, ingredients of such serum vary from lot to lot. However, such a variation does not occur in serum-free medium. Therefore, the serum-free cultured synovium-derived mesenchymal stem cells are excellent in safety and quality. It is also possible to minimize the risks such as biological contamination and mixing of an immunogenic substance, and to minimize an amount of a substance(s) that do not exist in a body. It is also easy to control the quality since biological materials that are contained are clear. Further, mesenchymal stem cells that are serum-free cultured in any of some certain serum-free media, such as STK (registered trademark) medium, have an excellent proliferation rate.

The term "serum-free culture" herein is intended to mean culture without use of serum. For example, the "serum-free culture" is intended to mean culture using a serum-free medium that does not contain serum. The term "low serum culture" is intended to mean culture using a medium that contains less serum than a general serum-containing medium (e.g., 10% FBS-containing medium) and culture using a serum-containing medium for a shorter period of time than general culture using a serum-containing medium.

(Serum-free culture)

The following description will discuss an example of a serum-free medium that is used for serum-free culture of the synovium-derived mesenchymal stem cells which form an artificial tissue contained in the intervertebral disc therapeutic agent. A basal medium for constituting the serum-free medium is not particularly limited, provided that the basal medium is a medium for animal cells which is well known in the present field. Preferred examples of the basal medium include Ham's F12 medium, DMEM medium, RPMI-1640 medium, and MCDB medium. One of these basal media may be used alone or a mixture of two or more of them may be used. In an embodiment, the basal medium for constituting the serum-free medium is preferably a medium obtained by mixing MCDB and DMEM at a ratio of 1:1.

In an embodiment, in culture of the synovium-derived mesenchymal stem cells, a serum-free medium that is obtained by adding FGF, PDGF, TGF-$\beta$, HGF, EGF, at least one phospholipid, and at least one fatty acid to the above basal medium may be used. The content of the FGF with respect to the basal medium is, at a final concentration, preferably 0.1 ng/ml to 100 ng/ml and more preferably 3 ng/ml. The content of the PDGF with respect to the basal medium is, at a final concentration, preferably 0.5 ng/ml to 100 ng/ml and more preferably 10 ng/ml. The content of the TGF-$\beta$ with respect to the basal medium is, at a final concentration, preferably 0.5 ng/ml to 100 ng/ml and more preferably 10 ng/ml.

The content of the HGF with respect to the basal medium is, at a final concentration, preferably 0.1 ng/ml to 50 ng/ml and more preferably 5 ng/ml. The content of EGF with respect to the basal medium is, at a final concentration, preferably 0.5 ng/ml to 200 ng/ml and more preferably 20 ng/ml. The total content of the phospholipid with respect to the basal medium is, at a final concentration, preferably 0.1 ng/ml to 30 pg/ml and more preferably 10 pg/ml. The total content of the fatty acid with respect to the basal medium is preferably 1/1000 to 1/10 of the basal medium, and more preferably 1/100 of the basal medium.

With use of such a serum-free medium, it is possible to obtain a proliferation-promoting effect that is equal to or greater than that of a serum-containing medium while preventing contamination with a foreign protein. Consequently, the synovium-derived mesenchymal stem cells can be proliferated as desired.

The serum-free medium may contain a phospholipid. Examples of the phospholipid include phosphatidic acid, lysophosphatidic acid, phosphatidylinositol, phosphatidylserine, phosphatidylethanolamine, phosphatidylcholine, and phosphatidylglycerol. It is possible to contain one of these phospholipids alone or two or more of them in combination. In an embodiment, the serum-free medium may contain a combination of phosphatidic acid and phosphatidylcholine. These phospholipids may also be derived from an animal or a plant.

The serum-free medium may contain a fatty acid. Examples of the fatty acid include linoleic acid, oleic acid, linolenic acid, arachidonic acid, myristic acid, palmitoyl acid, palmitic acid, and stearic acid. A medium additive according to the present embodiment may contain one of these fatty acids alone or two or more of them in combination. Further, the serum-free medium according to the present embodiment may contain cholesterol in addition to the fatty acid.

The "FGF" as used herein is intended to mean a growth factor selected from a fibroblast growth factor (FGF) family. The FGF is preferably FGF-2 (bFGF) but may be selected from another FGF family, such as FGF-1. The "PDGF" as used herein is intended to mean a growth factor selected from a platelet-derived growth factor (PDGF) family and is preferably PDGF-BB or PDGF-AB. Further, the "TGF-$\beta$" as used herein is intended to mean a growth factor selected from a transforming growth factor-$\beta$ (TGF-$\beta$) family. The TGF-$\beta$ is preferably TGF-$\beta$1, but may be selected from another TGF-$\beta$ family.

The "HGF" as used herein is intended to mean a growth factor selected from a hepatocyte growth factor family. The "EGF" as used herein is intended mean a growth factor selected from an epidermal growth factor (EGF) family.

In an embodiment, the serum-free medium may further contain at least two factors that are selected from the group consisting of a connective tissue growth factor (CTGF), a vascular endothelial growth factor (VEGF), and an ascorbic acid compound.

The "ascorbic acid compound" as used herein is intended to mean an ascorbic acid (vitamin C) or an ascorbic acid diphosphate, or a compound similar to these.

Note that the above growth factor contained in the serum-free medium may be a natural growth factor or may be produced by genetic modification.

In an aspect, the serum-free medium preferably contains a lipid antioxidant. In an embodiment, the lipid antioxidant contained in the serum-free medium can be DL-α-tocopherol acetate (vitamin E). Also, the serum-free medium may further contain a surfactant. In an embodiment, the surfactant contained in the serum-free medium can be Pluronic F-68 or Tween 80.

The serum-free medium may further contain insulin, transferrin and selenate. The "insulin" as used herein may be an insulin-like growth factor, or may be derived from natural cells or produced by genetic modification. The medium additive according to an embodiment of the present invention may further contain dexamethasone or another glucocorticoid.

In a case where the synovium-derived mesenchymal stem cells are serum-free cultured, mesenchymal stem cells that have been isolated by a conventionally known method from a synovial tissue of an animal such as a human are seeded in the above-described serum-free medium, and cultured until the cells are proliferated to a desired number. As culture conditions, it is preferable that: $1 \times 10^4$ to $2 \times 10^4$ mesenchymal stem cells be seeded per ml of the medium; and culture is carried out at a culture temperature of 37°C ± 1°C, for a culture time of 48 hours to 96 hours, and under $5\%CO_2$. When cultured in this manner, mesenchymal stem cells that maintain an immune suppression ability or that have an improved immune suppression ability can be efficiently obtained in large quantities.

A culture container used for the culture is not particularly limited, provided that in the culture container, the mesenchymal stem cells can proliferate. For example, a 75-cm$^2$ flask manufactured by Falcon or a 75-cm$^2$ flask manufactured by Sumitomo Bakelite may be suitably used. However, depending on cells, the proliferation of the cells may be affected by a type of the culture container. Therefore, in order to more efficiently proliferate the synovium-derived mesenchymal stem cells, it is preferable to use, for each type of mesenchymal stem cells to be proliferated (hereinafter, also referred to as "proliferation target cells"), a different culture container that is suitable for proliferation of the proliferation target cells.

The culture container suitable for proliferation of a proliferation target cell can be selected by, for example, making the proliferation target cell select an optimum culture container. Specifically, first, a plurality of types of culture containers are prepared, and proliferation target cells are proliferated under the same conditions except for types of culture containers. Further, the number of cells two weeks after the start of culture is counted by a known method. Then, it is possible to determine a culture container in which a larger number of cells are counted as a culture container which is more suitable for proliferation of the proliferation target cells. In a case where the proliferation rate of the proliferation target cells is high, it is possible to determine, before two weeks passes from the start of culture, a culture container in which the proliferation target cells take a shorter period of time before the number of the cells reaches 80% to 90% of that in a confluent state as a culture container which is more suitable for proliferation of the proliferation target cells.

Note that for proliferation of the mesenchymal stem cells, it is essential for cells to adhere to the culture container. Therefore, in a case where adhesion of the proliferation target cells to a culture container is weak, it is preferable to further contain a cell adhesion molecule in the serum-free medium when the proliferation target cells are serum-free cultured. The cell adhesion molecule can be, for example, fibronectin, collagen, or gelatin. It is possible to use one of these cell adhesion molecules alone or two or more in combination.

The content of the cell adhesion molecule with respect to the serum-free medium is, at a final concentration, preferably 1 pg/ml to 50 μg/ml, and more preferably 5 μg/ml. In an embodiment, in a case where fibronectin is used as the cell adhesion molecule, it is possible to improve adhesion efficiency of the proliferation target cells to a culture container by adding fibronectin such that the final concentration of the fibronectin with respect to the serum-free medium is 5 μg/ml.

In addition, in the serum-free culture, the synovium-derived mesenchymal stem cells may be passaged at least once. The mesenchymal stem cells proliferate in an anchorage-dependent manner. Therefore, in a case where the mesenchymal stem cells are locally and unevenly proliferated or the like, the culture conditions can be improved by passage of the synovium-derived mesenchymal stem cells during proliferation.

A passage method for the synovium-derived mesenchymal stem cells is not particularly limited. It is possible to passage the mesenchymal stem cells by a conventionally known passage method for mesenchymal stem cells. In view of a good condition of the synovium-derived mesenchymal stem cells after passage, in a case where passage is carried out, it is preferable to dissociate the mesenchymal stem cells with use of a cell dissociation agent that does not contain a component derived from a mammal or a microorganism. The above "cell dissociation agent that does not contain a component derived from a mammal or a microorganism" can be, for example, TrypLE Select CTS (Thermo Fisher Scientific Inc.) or ACCUTASE (Innovative Cell Technologies, Inc.).

(Artificial tissue)

An artificial tissue contained in the intervertebral disc therapeutic agent in accordance with an aspect of the present invention is a scaffold-free artificial tissue in which synovium-derived mesenchymal stem cells have formed a three-dimensional structure. The artificial tissue can be a tissue engineered construct (TEC). It has been reported that in a case where cells are transplanted by administering a cell suspension to an affected area, administered cells are likely to leave from a transplantation site and do not remain at the transplantation site. However, the intervertebral disc therapeutic agent in accordance with an aspect of the present invention contains a scaffold-free artificial tissue that is a three-dimensional structure. Therefore, engraftment of the cells to the transplantation site is easy and the intervertebral disc therapeutic agent can exert the therapeutic effect for a long period of time.

The scaffold-free artificial tissue that has formed a three-dimensional structure can be a scaffold-free artificial tissue obtained by processing a mass of mesenchymal stem cells into a three-dimensional structure by a conventionally known method. In a case where the term "three-dimensional structure" is used herein regarding an artificial tissue, the "three-dimensional structure" refers to an object in which a matrix is oriented in three dimensions, in which cells are three-dimensionally arrayed, and which expands in three-dimensional directions containing cells that maintain cell junctions and orientation.

For the artificial tissue, area, thickness, and strength, which are suitable for treatment of an intervertebral disc, should be set as appropriate, and a person skilled in the art can set, as appropriate, size of the artificial tissue. This size can be set according to an environment where the artificial tissue is to be transplanted. In the case of a small-sized artificial tissue, there is an advantage that it is possible to inject the artificial tissue into a body cavity with a needle. On the other hand, in the case of a large-sized artificial tissue, there is an advantage that a sufficient number of cells can be easily administered because the artificial tissue can be easily handled, for example, the artificial tissue can be held by tweezers at surgery.

In a case where the artificial tissue is to be transplanted, it is preferable that the artificial tissue has at least a certain size. Such a size is for example, not less than $1\ cm^2$, preferably not less than $2\ cm^2$, and more preferably not less than $3\ cm^2$, in terms of the area of the artificial tissue that has formed a three-dimensional structure. The size may be more preferably not less than $4\ cm^2$, not less than $5\ cm^2$, not less than $6\ cm^2$, not less than $7\ cm^2$, not less than $8\ cm^2$, not less than $9\ cm^2$, not less than $10\ cm^2$, not less than $15\ cm^2$, or not less than $20\ cm^2$, and for example, not more than $40\ cm^2$, not more than $30\ cm^2$ or less, or not more than $20\ cm^2$. However, the size is not limited to these. The area can be not more than $1\ cm^2$ or not less than $40\ cm^2$, depending on an application.

In a case where the size is expressed in volume of the artificial tissue, the size is preferably not less than $2\ mm^3$, and more preferably not less than $40\ mm^3$, and may also be, for example, not more than $40\ mm^3$ or not more than $20\ mm^3$. However, the size is not limited to these. The size may be not less than $2\ mm^3$.

Although a sufficient thickness of an implantable artificial tissue varies depending on a part subject to transplantation, a person skilled in the art can set, as appropriate, the thickness of the artificial tissue. The thickness can be set according to an environment where the artificial tissue is to be transplanted. The thickness of the artificial tissue is intended to be not less than 2 mm, more preferably not less than 3 mm, and even more preferably not less than 5 mm. In a case where the artificial tissue is applied to a cartilage, the thickness of the artificial tissue can be, for example, not less than 1 mm, preferably not less than 2 mm, more preferably not less than 3 mm, and even more preferably not less than 5 mm. Meanwhile, in any case, the thickness may be not more than 1 mm, not more than 10 mm, or not more than 5 mm.

The number of cells that constitute the artificial tissue may be selected as appropriate. The artificial tissue may be, for example, a mass of 50 to 200 cells, or a mass of 1 million to 100 million cells. The mass may also be a small mass or a large mass.

(Scaffold-free)

The term "scaffold-free" (without a base material) herein refers to containing substantially no material (base material, i.e., scaffold) that is conventionally used when an artificial tissue is produced. Examples of the material of such a scaffold can include chemical polymeric compounds, ceramics, and biologics such as polysaccharides, collagen, gelatin, and hyaluronic acid. However, the material is not limited to these. The scaffold means a material that is substantially solid and has strength with which the scaffold can support cells or tissue.

Conventionally, a "scaffold type" cell preparation has been mainly used. This cell preparation is obtained as a result of processing cells into a three-dimensional structure by artificially adding a material that serves as a scaffold for the cells and tissue, i.e., a scaffold so that the cells are caused to adhere or be held and can grow. Recently, however, the risk of artificially adding a material is concerned, and accordingly, "scaffold-free" cell preparations is being developed. The "scaffold-free" cell preparations are manufactured, without adding a scaffold, by a method in which for example, cells themselves are stimulated to produce, by the cells, an environment that serves as a scaffold for the cells.

In a case where the artificial tissue is a scaffold-free three-dimensional structure, it is possible to reduce the amount of a material other than mesenchymal stem cells that are contained in the intervertebral disc therapeutic agent. Further, in a case where the artificial tissue is a scaffold-free three-dimensional structure, the following can be realized: ingredients are known; the risks such as biological contamination and mixing of an immunogenic substance are minimized: and a substance that does not exist in a body is minimized. For example, a natural product such as collagen may be used as the scaffold. Ingredients of such a natural product vary from lot to lot. However, since the artificial tissue is a scaffold-free three-dimensional structure, ingredients are known, so that the artificial tissue is excellent in safety and quality stability. Further, the natural product described above is at the risks of biological contamination and mixing of an immunogenic substance. Such risks can be reduced in a case where the artificial tissue is a scaffold-free three-dimensional structure.

A method of obtaining a scaffold-free artificial tissue which is a three-dimensional structure containing the synovium-derived mesenchymal stem cells and which is contained in the intervertebral disc therapeutic agent in accordance with an aspect of the present invention can be, for example, a conventionally known method using a lowadhesion plate, a micropatterned surface plate, or the like, and a conventionally known hanging-drop method. Further, the artificial tissue may be prepared by using a method disclosed in Japanese Patent No. 4522994. It is also possible to use a commercially available substance. For example, gMSC (registered trademark) 1 (manufactured by Twocells Co., Ltd.) can be suitably used.

(Extracellular matrix)

The intervertebral disc therapeutic agent in accordance with an aspect of the present invention may further contain an extracellular matrix derived from mesenchymal stem cells. The term "extracellular matrix" herein means a substance that is also called an intercellular matrix and that exists between somatic cells regardless of epidermal cells or non-epidermal cells.

The extracellular matrix is known to be one of biological materials that are produced by cells and also known to be involved in constituting an internal environment necessary for survival of all somatic cells as well as supporting tissue. Examples of a representative extracellular matrix include, but are not limited to, collagen, elastin, vitronectin, fibronectin, laminin, thrombospondin, proteoglycans (e.g., decorin, biglycan, fibromodulin, lumican, hyaluronic acid, and aggrecan). A variety of extracellular matrices may be used in an embodiment of the present invention provided that the extracellular matrix is responsible for cell adhesion.

The extracellular matrix preferably includes at least one selected from the group consisting of collagen, vitronectin, and fibronectin. In the intervertebral disc therapeutic agent, the extracellular matrix may be integrated with the artificial tissue to form the three-dimensional structure, or the extracellular matrix may be present in the intervertebral disc therapeutic agent independently of the artificial tissue. The intervertebral disc therapeutic agent may further include a reagent, buffer, or the like for stably holding the artificial tissue.

(Method of treating intervertebral disc)

A method of treating an intervertebral disc in accordance with an aspect of the present invention includes the step of administering a scaffold-free artificial tissue in which synovium-derived mesenchymal stem cells have formed a three-dimensional structure. The mesenchymal stem cells that form an artificial tissue that is to be administered in the method of treating an intervertebral disc may be serum-free cultured cells. Moreover, the mesenchymal stem cells that form the artificial tissue that is to be administered in the method of treating an intervertebral disc may be derived from human synovium. The artificial tissue that is to be administered in the method of treating an intervertebral disc may further contain an extracellular matrix derived from mesenchymal stem cells. In other words, an aspect of the artificial tissue in the method of treating an intervertebral disc is an artificial tissue contained in the intervertebral disc therapeutic agent in accordance with an aspect of the present invention. Therefore, for an explanation of the artificial tissue, an explanation of the artificial tissue in the intervertebral disc therapeutic agent is incorporated.

In the step of administering, the artificial tissue can be administered by, for example, transplanting the artificial tissue to a treatment site or injecting, into a treatment site, an injection which contains the artificial tissue. In the step of administering, the artificial tissue may be administered alone or in combination with another therapeutic agent.

A dose of the artificial tissue administered in the step of administering can be determined, as appropriate, by a person skilled in the art, in consideration of, for example, the following: a treatment purpose; a target disease (e.g., type and severity); age, weight, sex, medical history of a patient; and/or a form or type of tissue. A frequency of administration of the artificial tissue in the step of administering can also be determined, as appropriate, by a person skilled in the art, in consideration of, for example, the following: a treatment purpose; a target disease (e.g., type and severity); and age, weight, sex, medical history, and a course of treatment of a patient. The frequency of administration may be, for example, daily to once a month (e.g., once a week to once a month). The dose of the artificial tissue administered and the frequency of administration of the artificial tissue in the step of administering may be adjusted, as appropriate, in accordance with the course of treatment.

The treatment site at which the artificial tissue is administered in the step of administering is a damaged site or a defective site of an intervertebral disc. In a case where the artificial tissue is administered to the damaged site or the defective site of the intervertebral disc, regeneration of the intervertebral disc is promoted. Thus, it is possible to treat damage or deficiency of the intervertebral disc.

(Mesenchymal stem cells for use in treatment of intervertebral discs)

Mesenchymal stem cells in accordance with an aspect of the present invention are mesenchymal stem cells for use in treatment of an intervertebral disc. The mesenchymal stem cells are synovium-derived and form a scaffold-free artificial tissue which is a three-dimensional structure. The mesenchymal stem cells for use in the treatment of an intervertebral disc may be serum-free cultured cells. Moreover, the mesenchymal stem cells for use in treatment of the intervertebral disc may be derived from human synovium. The mesenchymal stem cells for use in treatment of an intervertebral disc may further contain an extracellular matrix derived from mesenchymal stem cells. That is, the mesenchymal stem cells for use in treatment of an intervertebral disc are mesenchymal stem cells that form the artificial tissue which is included in the intervertebral disc therapeutic agent in accordance with an aspect of the present invention. Therefore, for an explanation of the mesenchymal stem cells, an explanation of the mesenchymal stem cells and the artificial tissue in the intervertebral disc therapeutic agent is incorporated.

The mesenchymal stem cells for use in treatment of an intervertebral disc can be used for treatment of a damaged site or a defective site of the intervertebral disc. The mesenchymal stem cells for use in treatment of an intervertebral disc treat damage or deficiency of the intervertebral disc, when administered to a site that requires treatment. When the mesenchymal stem cells for use in treatment of an intervertebral disc are administered to a damaged site or a defective site of the intervertebral disc, regeneration of the intervertebral disc at an administration site is promoted. Thus, it is possible to treat damage or deficiency of the intervertebral disc.

(Use of mesenchymal stem cells for manufacturing intervertebral disc therapeutic drug)

Use of mesenchymal stem cells for manufacturing an intervertebral disc therapeutic drug in accordance with an aspect of the present invention is use of the mesenchymal stem cells for manufacturing an intervertebral disc therapeutic drug. The mesenchymal stem cells are synovium-derived and form a scaffold-free artificial tissue which is a three-dimensional structure. In the use of the mesenchymal stem cells for manufacturing the intervertebral disc therapeutic drug, the mesenchymal stem cells may be serum-free cultured cells. Further, in the use of the mesenchymal stem cells for manufacturing the intervertebral disc therapeutic drug, the mesenchymal stem cells may be derived from human synovium. In the use of the mesenchymal stem cells for manufacturing the intervertebral disc therapeutic drug, an artificial tissue may further contain an extracellular matrix derived from the mesenchymal stem cells. In other words, an aspect of the artificial tissue in the use of the mesenchymal stem cells for manufacturing the intervertebral disc therapeutic drug is an artificial tissue contained in the intervertebral disc therapeutic agent in accordance with an aspect of the present invention. Therefore, for an explanation of the artificial tissue, an explanation of the artificial tissue in the intervertebral disc therapeutic agent is incorporated.

The intervertebral disc therapeutic drug manufactured by use of the mesenchymal stem cells can further include a pharmaceutically acceptable carrier or the like together with a scaffold-free artificial tissue in which synovium-derived mesenchymal stem cells have formed a three-dimensional structure. The pharmaceutically acceptable carrier of the intervertebral disc therapeutic drug includes any substance known in the present field. Examples of the pharmaceutically acceptable carrier include, but are not limited to, antioxidants, preservatives, colorants, flavorings, diluents, emulsifiers, suspensions, solvents, fillers, extenders, buffers, delivery vehicles, diluents, excipients, and/or pharmaceutical adjuvants.

The intervertebral disc therapeutic drug manufactured with use of mesenchymal stem cells can be used for treatment of a damaged site or a defective site. The intervertebral disc therapeutic drug treats damage or deficiency of the intervertebral disc, when administered to a site that requires treatment. When the intervertebral disc therapeutic drug is administered to a damaged site or a defective site of an intervertebral disc, regeneration of the intervertebral disc at an administration site is promoted. Thus, the intervertebral disc therapeutic agent can treat damage or deficiency of the intervertebral disc.

Aspects of the present invention can also be expressed as follows:
An intervertebral disc therapeutic agent in accordance with Aspect 1 of the present invention contains a scaffold-free artificial tissue in which synovium-derived mesenchymal stem cells have formed a three-dimensional structure.

An intervertebral disc therapeutic agent in accordance with Aspect 2 of the present invention may be configured such that in Aspect 1, the mesenchymal stem cells are serum-free cultured cells.

An intervertebral disc therapeutic agent in accordance with Aspect 3 of the present invention may be configured such that in Aspect 1 or 2, the mesenchymal stem cells are derived from human synovium.

An intervertebral disc therapeutic agent in accordance with Aspect 4 of the present invention may be configured to further contain, in any one of Aspects 1 to 3, an extracellular matrix derived from the mesenchymal stem cells.

A method for treating an intervertebral disc in accordance with Aspect 5 of the present invention includes the step of administering a scaffold-free artificial tissue in which synovium-derived mesenchymal stem cells have formed a three-dimensional structure.

A method for treating an intervertebral disc in accordance with Aspect 6 of the present invention may be configured such that in Aspect 5, the mesenchymal stem cells are serum-free cultured cells.

A method for treating an intervertebral disc in accordance with Aspect 7 of the present invention may be configured such that in Aspect 5 or 6, the mesenchymal stem cells are derived from human synovium.

A method for treating an intervertebral disc in accordance with Aspect 8 of the present invention may be configured to such that, in any one of Aspects 5 to 7, the artificial tissue further contains an extracellular matrix derived from the mesenchymal stem cells.

Mesenchymal stem cells in accordance with Aspect 9 of the present invention are mesenchymal stem cells for use in treatment of an intervertebral disc, the mesenchymal stem cells being synovium-derived and forming a scaffold-free artificial tissue which is a three-dimensional structure.

Mesenchymal stem cells in accordance with Aspect 10 of the present invention may be configured to be, in Aspect 9, serum-free cultured cells.

Mesenchymal stem cells in accordance with Aspect 11 of the present invention may be configured to be, in Aspect 9 or 10, derived from human synovium.

Mesenchymal stem cells in accordance with Aspect 12 of the present invention may be configured to further contain, in any one of Aspects 9 to 11, an extracellular matrix derived from the mesenchymal stem cells.

Use of mesenchymal stem cells in accordance with Aspect 13 of the present invention is use of mesenchymal stem cells for manufacturing an intervertebral disc therapeutic drug, the mesenchymal stem cells being synovium-derived and forming a scaffold-free artificial tissue which is a three-dimensional structure.

Use of mesenchymal stem cells in accordance with Aspect 14 of the present invention is may be configured such that, in Aspect 13, the mesenchymal stem cells are serum-free cultured cells.

Use of mesenchymal stem cells in accordance with Aspect 15 of the present invention is may be configured such that, in Aspect 13 or 14, the mesenchymal stem cells are derived from human synovium.

Use of mesenchymal stem cells in accordance with Aspect 16 of the present invention is may be configured such that, in any one of Aspects 13 to 15, the artificial tissue further contains an extracellular matrix derived from the mesenchymal stem cells.

Examples

(Comparison of intervertebral disc therapeutic effect between human synovium gMSC (registered trademark) 1 and rat synovium TEC)

(Transplantation of gMSC (registered trademark) 1 and rat synovium TEC)

Male 10-week-old SD rats were used in this experiment. As a scaffold-free artificial tissue in which serum-free cultured human synovium-derived mesenchymal stem cells had formed a three-dimensional structure, gMSC (registered trademark) 1 (manufactured by Twocells Co., Ltd.) was used. As a scaffold-free artificial tissue in which rat synovium-derived mesenchymal stem cells had formed a three-dimensional structure, a rat synovium TEC, which was prepared with use of rat synovium-derived mesenchymal stem cells by a method disclosed in Japanese Patent No. 4522994.

An approximately 1-cm longitudinal skin incision was made on a dorsal side of a rat to expose an annulus fibrosus. Then, the annulus fibrosus was incised longitudinally and all internal nucleus pulposus was extracted. The gMSC (registered trademark) 1 was transplanted into a nucleus pulposus extraction site. Then, each of the following groups were prepared: a nucleus pulposus extraction group in which the annulus fibrosus and skin were closed without transplantation; a nucleus pulposus replantation group in which the nucleus pulposus having been extracted was replanted and the annulus fibrosus and skin were closed; and a TEC group in which a rat synovium TEC was transplanted. Also prepared was a Sham group in which only skin was incised and an annulus fibrosus was exposed and in which nucleus pulposus was not extracted.

(Evaluation of intervertebral disc height and CT score)

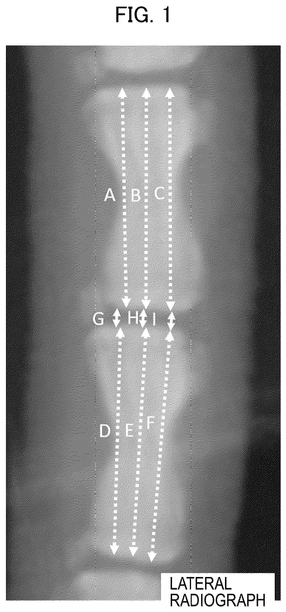

Six weeks after the transplantation, rats were anesthetized with use of a triple anesthetic, and lateral radiographs of caudal vertebrae were taken. The intervertebral disc height was evaluated with use of a disk height indices (DHIs). The DHIs each were calculated by measuring sites illustrated in Fig. 1 according to the following formula 1.

$$DHI = \frac{2 \times (G + H + I)}{A + B + C + D + E + F} \quad \dots [\text{Formula 1}]$$

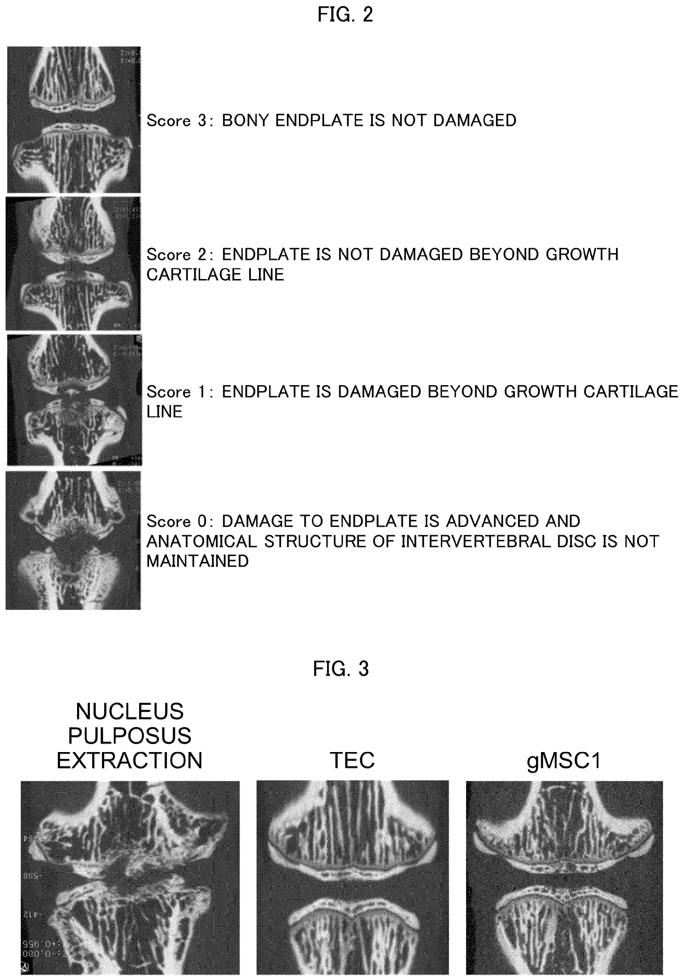

After rats were euthanized, an intervertebral disc of a caudal vertebra of each of the rats having undergone surgery were extracted. Then, an Ex vivo CT image was taken and endplate damage was evaluated by a CT score. The CT score was evaluated by criteria shown in Fig. 2.

(Human vimentin immunostaining of rat intervertebral disc)

A paraffin tissue section (5 μm thick) was deparaffined. Then, antigen retrieval was carried out with use of EDTA (AbcamTris-EDTA buffer ab93684, 80 °C, and 15 min). In addition, a primary antibody was reacted with an Abcam Anti-Vimentin antibody (ab16700, 200 fold dilution, and at room temperature for 1 h), and a secondary antibody was reacted with Simple Stain Rat MAX-PO (NICHIREI BIOSCIENCES, at room temperature for 30 min). As a result, DAB color was developed (at room temperature for 5 min). Nuclear staining was carried out with a hematoxylin solution. Then, encapsulation was carried out.

(Results)

Fig. 3 shows radiographs of surgery sites in the nucleus pulposus extraction group, the TEC group, and the gMSC (registered trademark) 1 group. As illustrated in Fig. 3, in the gMSC (registered trademark) 1 group, endplate degeneration was suppressed as in a manner similar to that of the TEC group.

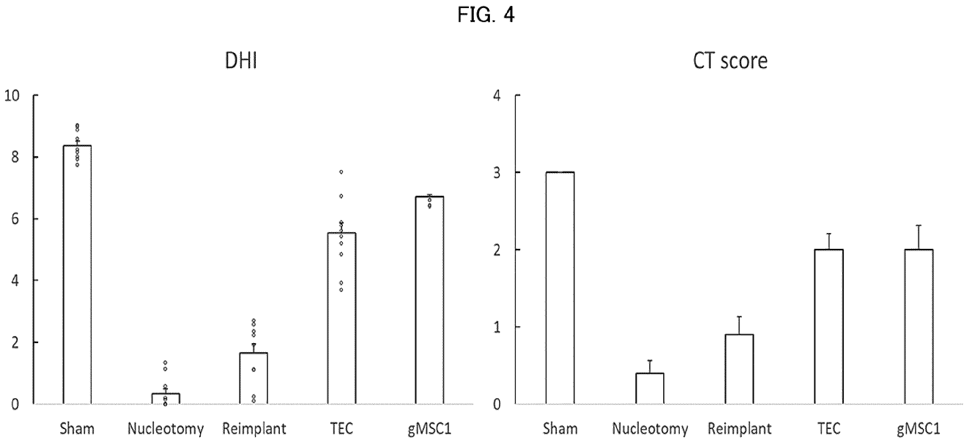

Fig. 4 shows the DHI and the CT score of each of the above groups. As illustrated in Fig. 4, in the TEC group and the gMSC (registered trademark) 1 group, the intervertebral disc height was maintained and the endplate damage score was also good. The gMSC (registered trademark) 1 group was superior to the TEC group at least in an effect of maintaining the intervertebral disc height.

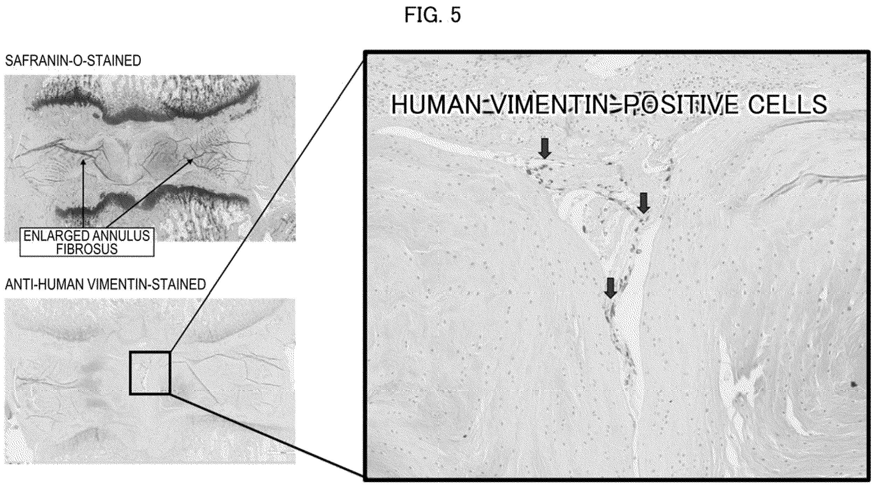

Fig. 5 shows a vimentin staining result of the MSC (registered trademark) 1 group. As illustrated in Fig. 5, at 6 weeks after the surgery, a thickened annulus fibrosus was observed, and human vimentin-positive cells were observed. Survival of cells derived from the gMSC (registered trademark) 1 were also confirmed at 6 weeks after the surgery, and engraftment was favorable. Further, enlargement of a surrounding annulus fibrosus and an effect of maintaining the intervertebral disc structure were demonstrated.

[Additional Remarks]

The present invention is not limited to the above embodiments or Examples. The present invention may be altered within the scope of the claims by a person skilled in the art. The present invention also encompasses, in its technical scope, any embodiment derived by combining technical means disclosed in differing embodiments or Examples.

Industrial Applicability

Use of an embodiment of the present invention can provide a transplantation therapeutic agent that uses mesenchymal stem cells and that is highly useful. Therefore, an embodiment of the present invention can be suitably applied to regenerative medicine such as transplantation treatment using mesenchymal stem cells.

**Claims**

1. An intervertebral disc therapeutic agent containing a scaffold-free artificial tissue in which synovium-derived mesenchymal stem cells have formed a three-dimensional structure.

2. The intervertebral disc therapeutic agent according to claim 1, wherein the mesenchymal stem cells are serum-free cultured cells.

3. The intervertebral disc therapeutic agent according to claim 1 or 2, wherein the mesenchymal stem cells are derived from human synovium.

4. The intervertebral disc therapeutic agent according to claim 1 or 2, further containing an extracellular matrix derived from the mesenchymal stem cells.

FIG. 1

LATERAL
RADIOGRAPH

## FIG. 2

Score 3: BONY ENDPLATE IS NOT DAMAGED

Score 2: ENDPLATE IS NOT DAMAGED BEYOND GROWTH CARTILAGE LINE

Score 1: ENDPLATE IS DAMAGED BEYOND GROWTH CARTILAGE LINE

Score 0: DAMAGE TO ENDPLATE IS ADVANCED AND ANATOMICAL STRUCTURE OF INTERVERTEBRAL DISC IS NOT MAINTAINED

## FIG. 3

NUCLEUS PULPOSUS EXTRACTION          TEC          gMSC1

FIG. 4

## DHI

## CT score

EP 4 591 869 A1

FIG. 5

SAFRANIN-O-STAINED

ENLARGED ANNULUS FIBROSUS

ANTI-HUMAN VIMENTIN-STAINED

HUMAN-VIMENTIN-POSITIVE CELLS

EP 4 591 869 A1

## INTERNATIONAL SEARCH REPORT

| International application No. |
|---|
| **PCT/JP2023/033663** |

| A. | CLASSIFICATION OF SUBJECT MATTER |
|---|---|

*A61K 35/32*(2015.01)i; *A61P 19/08*(2006.01)i; *A61P 43/00*(2006.01)i; *C12N 5/0775*(2010.01)i
FI:    A61K35/32; A61P19/08; A61P43/00 107; C12N5/0775

According to International Patent Classification (IPC) or to both national classification and IPC

| B. | FIELDS SEARCHED |
|---|---|

Minimum documentation searched (classification system followed by classification symbols)

A61K35/32; A61P19/08; A61P43/00; C12N5/0775

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | 海渡貴司ほか, 間葉系幹細胞から作成した三次元複合体組織による椎間板再生, 日本整形外科学会雑誌, 2021, vol. 95, no. 8, p. S1607 (2-4-S14-3) text, (KAITO, Takashi et al. The Journal of Japanese Orthopaedic Surgical Society.), non-official translation (Intervertebral disc regeneration using three-dimensional composite tissue generated from mesenchymal stem cells) | 1, 3, 4 |
| Y | | 2-4 |
| X | 海渡貴司ほか, 間葉系幹細胞由来三次元複合体移植による椎間板再生, 日本整形外科学会雑誌, 15 September 2022, vol. 96, no. 8, p. S1611 (1-8-S11-2) text, (KAITO, Takashi et al. The Journal of Japanese Orthopaedic Surgical Society.), non-official translation (Intervertebral disc regeneration by mesenchymal stem cell-derived three-dimensional composite implant) | 1, 3, 4 |
| Y | | 2-4 |

☑ Further documents are listed in the continuation of Box C.          ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **22 November 2023** | **05 December 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

International application No.

**PCT/JP2023/033663**

**C.    DOCUMENTS CONSIDERED TO BE RELEVANT**

| Category* | Citation of document, with indication, where appropriate, of the relevant passages | Relevant to claim No. |
|---|---|---|
| Y | 無血清培養法により製造した同種滑膜間葉系幹細胞由来三次元人工組織の薬事承認申請に資する非臨床試験, 厚生労働科学研究成果データベース [オンライン], 09 July 2018, [retrieval date 20 November 2023], Internet: <URL:https://mhlw-grants.niph.go.jp/project/24674><br>          Research method, Results and reviews, Result, (MHLW Grants System [online]), non-official translation (Nonclinical tests contributing to pharmaceutical approval application of three-dimensional artificial tissue derived from allogenic synovial mesenchymal stem cells manufactured using a serum-free culture method) | 2-4 |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005012512 A

- JP 4522994 B

**Non-patent literature cited in the description**

- **H. ISHIGURO**. *Acta Biomaterialia*, 2019, vol. 87, 118-129